(19) Europäisches Patentamt / European Patent Office / Office européen des brevets

(11) EP 2 707 404 B1

(12) FASCICULE DE BREVET EUROPEEN

(45) Date de publication et mention de la délivrance du brevet:
**30.09.2015 Bulletin 2015/40**

(21) Numéro de dépôt: **12728665.6**

(22) Date de dépôt: **11.05.2012**

(51) Int Cl.:
***C08F 293/00*** (2006.01) ***A61K 8/90*** (2006.01)
***A61Q 1/02*** (2006.01) ***A61Q 1/04*** (2006.01)
***A61Q 1/10*** (2006.01) ***A61Q 17/04*** (2006.01)
***A61Q 19/00*** (2006.01) ***A61Q 19/04*** (2006.01)
***A61Q 3/02*** (2006.01) ***C08F 20/10*** (2006.01)
***A61Q 5/00*** (2006.01)

(86) Numéro de dépôt international:
**PCT/FR2012/051055**

(87) Numéro de publication internationale:
**WO 2012/156630 (22.11.2012 Gazette 2012/47)**

# (54) POLYMÈRE SÉQUENCÉ COMPRENANT DE L'ACRYLATE D'ISOBUTYLE ET DE L'ACIDE ACRYLIQUE, COMPOSITION COSMÉTIQUE ET PROCÉDÉ DE TRAITEMENT

BLOCKPOLYMER MIT ISOBUTYLACRYLAT UND ACRYLSÄURE, KOSMETISCHE ZUSAMMENSETZUNG UND BEHANDLUNGSVERFAHREN

BLOCK POLYMER INCLUDING ISOBUTYL ACRYLATE AND ACRYLIC ACID, COSMETIC COMPOSITION AND TREATMENT METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.05.2011 FR 1154161**
**19.05.2011 US 201161487751 P**

(43) Date de publication de la demande:
**19.03.2014 Bulletin 2014/12**

(73) Titulaire: **L'OREAL**
**75008 Paris (FR)**

(72) Inventeurs:
- **FARCET, Céline**
  **F-75012 Paris (FR)**
- **LION, Bertrand**
  **F-75012 Paris (FR)**

(74) Mandataire: **Kromer, Christophe**
**L'Oréal**
**D.I.P.I.**
**25-29 Quai Aulagnier**
**92665 Asnières-sur-Seine Cedex (FR)**

(56) Documents cités:
**EP-A1- 2 147 940** **EP-A1- 2 147 941**
**FR-A1- 2 949 675** **FR-A1- 2 949 677**
**US-A1- 2011 020 263**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne de nouveaux polymères séquencés, ainsi que les compositions cosmétiques les comprenant, et un procédé de traitement cosmétique les employant.

Les polymères séquencés, aussi appelés à blocs, véhiculables dans un milieu non aqueux, notamment solubles ou dispersés dans ledit milieu, présentent des propriétés filmogènes intéressantes mais peuvent être pénalisés dans des applications dans le domaine du maquillage, notamment de par leurs sensibilités aux corps gras, tels que les huiles alimentaires ou le sébum.

La présente invention a pour objectif de proposer des polymères séquencés peu sensibles aux corps gras, notamment aux huiles alimentaires et au sébum.

On a en effet constaté que la présence de certains monomères au sein d'une même séquencé, lié à une configuration particulière du polymère (enchainement des séquences) pouvait permettre d'atteindre cet objectif.

La présente invention a donc pour objet un polymère di ou tri-séquencé de structure AB, ABA ou BAB, comprenant au moins une séquence A qui comprend de l'acrylate d'isobutyle et de l'acide acrylique.

L'invention concerne également une composition cosmétique comprenant, dans un milieu cosmétiquement acceptable, au moins un tel polymère.

Les polymères selon l'invention sont de préférence filmogènes et peu ou pas sensibles aux huiles alimentaires, donc susceptibles d'être employés avantageusement dans des compositions de maquillage.

Par filmogène, on entend un polymère apte à former, à lui seul ou en présence d'un agent auxiliaire de filmification, un dépôt continu sur un support, notamment sur les matières kératiniques.

Les polymères selon l'invention présentent par ailleurs une bonne tenue à l'huile d'olive (food proof), améliorées par rapport aux polymères séquencés connus.

Le polymère séquencé selon l'invention est de structure choisie parmi les structures suivantes : AB, BA et BAB.

Il peut donc comprendre deux séquences distinctes et donc être un polymère dibloc que l'on peut symboliser AB, comprenant une séquence A et une séquence B. Il peut également comprendre trois séquences distinctes et donc être un polymère tribloc que l'on peut symboliser soit ABA (ou ABA') lorsqu'il comprend une séquence centrale B et deux séquences externes A (ou A et A'), identiques ou non, mais généralement identiques; soit BAB (ou BAB') lorsqu'il comprend une séquence centrale A et deux séquences externes B (ou B et B'), identiques ou non, mais généralement identiques.

De préférence, il s'agit d'un polymère éthylénique, c'est-à-dire un polymère obtenu par polymérisation de monomères comprenant une insaturation éthylénique, de préférence une seule insaturation éthylénique par monomère.

De préférence, le polymère séquencé est linéaire (notamment sans ajout volontaire de réticulant et/ou de monomère difonctionnel).

Le polymère séquencé selon l'invention se caractérise notamment par le fait qu'il comprend une séquence (appelée ci-après séquence A) qui comprend à la fois des motifs acrylate d'isobutyle et acide acrylique.

De préférence, l'acrylate d'isobutyle représente 75 à 99,5% en poids, notamment 78 à 95% en poids du poids total du mélange acrylate d'isobutyle + acide acrylique. De préférence, l'acide acrylique représente 0,5 à 25% en poids, notamment 5 à 22% en poids du poids total du mélange acrylate d'isobutyle + acide acrylique.

De préférence, le mélange acrylate d'isobutyle + acide acrylique représente 50 à 100% en poids, notamment 60 à 90% en poids, voire 70 à 80% en poids, du poids total de ladite séquence.

Cette séquence A peut donc comprendre de monomères additionnels, qui peuvent être présents à raison de 0 à 50% en poids, notamment 10 à 40% en poids, voire 20 à 30% en poids, dans ladite séquence.

Ces monomères additionnels sont de préférence choisis parmi, seul ou en mélange, les monomères suivants ainsi que leurs sels :

(i) les (méth)acrylates de formule $CH_2$=CHCOOR ou $CH_2$=C($CH_3$)COOR dans laquelle R représente :

- un groupe alkyle linéaire ou ramifié, comprenant 1 à 30 atomes de carbone dans lequel se trouve(nt) éventuellement intercalé(s) un ou plusieurs hétéroatomes choisis parmi O, N, S et P et/ou ledit groupe alkyle pouvant être éventuellement substitué par un ou plusieurs substituants choisis parmi -OH, les atomes d'halogène (Cl, Br, I et F), les groupes -NR4R5, où R4 et R5 identiques ou différents représentent l'hydrogène ou un groupe alkyle linéaire ou ramifié, en C1-C6 ou un groupe phényle; et/ou les groupes polyoxyalkylènes, notamment polyoxyé-thylène et/ou polyoxypropylène, ledit groupe polyoxyalkylène étant constitué par la répétition de 5 à 30 motifs oxyalkylène;
- un groupe cycloalkyle en C3-C12, ledit groupe cycloalkyle pouvant comporter dans sa chaîne un ou plusieurs hétéroatomes choisis parmi O, N, S et/ou P, et/ou pouvant être éventuellement substitué par un ou plusieurs substituants choisis parmi -OH et les atomes d'halogène (Cl, Br, I et F);
- un groupe aryle en C4-C20 ou aralkyle en C5-C30 (groupe alkyle en C1-C8);

à l'exclusion de l'acrylate d'isobutyle;

(ii) les (méth)acrylamides de formule $CH_2$=$CHCONR_1R_2$ ou $CH_2$=$C(CH_3)CONR_1R_2$ dans laquelle R1 et R2, identiques ou différents, représentent H ou :

- un groupe alkyle, linéaire ou ramifié, comportant de 1 à 18 atomes de carbone, dans lequel se trouve(nt) éventuellement intercalé(s) un ou plusieurs hétéroatomes choisis parmi O, N, S et P; ledit groupe alkyle pouvant être substitué par un ou plusieurs substituants choisis parmi OH et les atomes d'halogène (Cl, Br, I et F), et/ou pouvant être interrompu par un groupe SiR4R5, où R4 et R5 identiques ou différents représentent un groupe alkyle en C1-C6 ou un groupe phényle;
  et notamment R1 et/ou R2 peuvent être un groupe méthyle, éthyle, propyle, isopropyle, n-butyle, isobutyle, tertiobutyle, hexyle, isohexyle, cyclohexyle, éthylhexyle, octyle, isooctyle, décyle, isodécyle, cyclodécyle, dodécyle, cyclododécyle, isononyle, lauryle, t-butylcyclohexyle, stéaryle; éthyl-2-perfluorohexyle; ou un groupe hydroxyalkyle en C1-4 tel que 2-hydroxyéthyle, 2-hydroxybutyle et 2-hydroxypropyle; ou un groupe alcoxy(C1-4)-alkyle(C1-4) tel que méthoxyéthyle, éthoxyéthyle et méthoxy-propyle,
- un groupe cycloalkyle en C3-C12, tel que isobornyle, ou un groupe hétérocycloalkyle (alkyle en C1-C4), tel que furfurylméthyle ou tétrahydrofurfurylméthyle,
- un groupe aryle en C4-C20 tel que le groupe phényle,
- un groupe aralkyle en C5-C30 (groupe alkyle en C1-C8) tel que 2-phényl éthyle, t-butylbenzyle ou benzyle,

(iii) les monomères à insaturation(s) éthylénique(s) comprenant au moins une fonction acide carboxylique, phosphorique ou sulfonique, ou anhydride, comme par exemple l'acide méthacrylique, l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylamidopropanesulfonique; et les sels de ceux ci; à l'exclusion de l'acide acrylique;

(iv) les éthers de vinyle de formule $R_6O$-CH=$CH_2$ ou les esters de vinyle de formule : $R_6$-COO-CH=$CH_2$ dans laquelle $R_6$ représente un groupe alkyle linéaire ou ramifié, comprenant de 1 à 22 atomes, ou un groupe alkyle cyclique comportant de 3 à 6 atomes de carbone et/ou un groupe aromatique, par exemple de type benzénique, anthracénique, et naphtalénique;

(v) les composés vinyliques de formules $CH_2$=CH-R9, $CH_2$=CH-$CH_2$-R9 ou $CH_2$=$C(CH_3)$-$CH_2$-R9 dans lesquelles R9 est :

- un groupe hydroxyle, halogène (Cl ou F), $NH_2$, acétamide ($NHCOCH_3$);
- un groupe OCOR11 où R11 représente un groupe alkyle en C2-C12, linéaire ou ramifié;
- un groupe OR10 où R10 représente un groupe phényle ou un groupe alkyle en C1-C12;
- un groupe alkyle linéaire ou ramifié, en C1-C18, dans lequel se trouve(nt) éventuellement intercalé(s) un ou plusieurs hétéroatomes choisis parmi O, N, S et P; ledit groupe alkyle pouvant être substitué par un ou plusieurs substituants choisis parmi OH et les atomes d'halogène (Cl, Br, I et F), et/ou pouvant être interrompu par un groupe SiR4R5, où R4 et R5 identiques ou différents représentent un groupe alkyle en C1-C6 ou un groupe phényle;
- un groupe cycloalkyle en C3-C12 tel que isobornyle, cyclohexane,
- un groupe aryle en C3-C20 tel que phényle,
- un groupe aralkyle en C4-C30 (groupe alkyle en C1-C8) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétrahydrofurfurylméthyle,

(vi) le styrène et ses dérivés, notamment tels que le méthylstyrène, le chlorostyrène ou le chlorométhylstyrène;

(vii) les monomères à insaturation éthylénique comprenant un ou plusieurs atomes de silicium tels que le méthacryloxypropyl triméthoxy silane, le méthacryloxypropyl tris(triméthylsiloxy)silane.

(viii) les macromonomères carbonés ou siliconés ayant au moins un groupe terminal polymérisable.

Il s'agit de tout polymère, notamment oligomère, comportant sur une seule de ses extrémités un groupe terminal, notamment polymérisable, apte à réagir lors de la réaction de polymérisation avec les monomères considérés, pour

former les chaînes latérales du polymère; ledit groupe terminal peut être avantageusement un groupe à insaturation éthylénique susceptible de se polymériser par voie radicalaire avec les monomères constituant le squelette. Ledit macromonomère permet de former les chaînes latérales du copolymère. Le groupe polymérisable du macromonomère peut être avantageusement un groupe à insaturation éthylénique susceptible de se polymériser par voie radicalaire. Ledit groupe terminal polymérisable peut être en particulier un groupe vinyle ou (méth)acrylate. Parmi les macromonomères additionnels susceptibles d'être employés, on peut notamment citer, seuls ou en mélange, ainsi que leurs sels, :

(a) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate; les macromonomères de poly(acrylate de stéaryle) ou de poly (méthacrylate de stéaryle) à extrémité mono(méth)acrylate. De tels macromonomères sont notamment décrits dans les brevets EP895467 et EP96459.

-(b) les polyoléfines ayant un groupe terminal à insaturation éthylénique, en particulier ceux ayant un groupement terminal (méth)acrylate. Comme exemple de telles polyoléfines, on peut citer en particulier les macromonomères suivants, étant entendu qu'ils ont un groupe terminal (méth)acrylate: les macromonomères de polyéthylène, les macromonomères de polypropylène, les macromonomères de copolymère polyéthylène/polypropylène, les macromonomères de copolymère polyéthylène/polybutylène, les macromonomères de polyisobutylène; les macromonomères de polybutadiène; les macromonomères de polyisoprène; les macromonomères de polybutadiène; les macromonomères de poly(éthylène/butylène)-polyisoprène. De tels macromonomères sont en particulier décrits dans US 5,625,005 qui mentionne des macromonomères éthylène/butylène et éthylène/propylène à groupement terminal réactif (méth)acrylate. On peut en particulier citer le méthacrylate de poly(éthylène/butylène), tel que celui commercialisé sous la dénomination Kraton Liquid L-1253 par Kraton Polymers.

-(c) les polydiméthylsiloxanes à groupement terminal mono(méth)acrylate, et notamment ceux de formule (IIa) suivante :

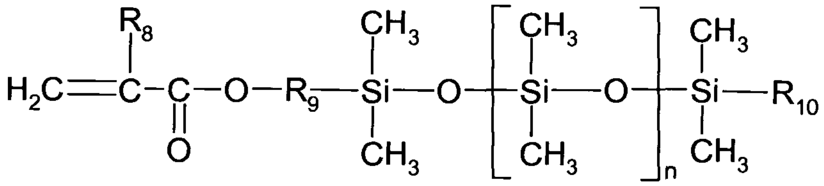

(IIa)

dans laquelle :

- $R_8$ désigne un atome d'hydrogène ou un groupement méthyle; de préférence méthyle;
- $R_9$ désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
- $R_{10}$ désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

Comme macromonomères siliconés, on peut en particulier citer les monométhacryloyloxypropyl polydiméthylsiloxanes tels que ceux commercialisés sous la dénomination PS560-K6 par UCT (United Chemical Technologies Inc.) ou sous la dénomination MCR-M17 par Gelest Inc.

Parmi ces monomères additionnels, on peut tout particulièrement citer :

- les (méth)acrylates en C1-C18, comprenant éventuellement un hétéroatome, et notamment de méthyle, éthyle, propyle, isopropyle, n-butyle, isobutyle (méthacrylate uniquement), tertiobutyle, hexyle, éthylhexyle, octyle, lauryle, isooctyle, isodécyle, dodécyle, cyclohexyle, t-butylcyclohexyle, stéaryle, éthyl-2-perfluorohexyle, 2-hydroxyéthyle, 2-hydroxybutyle, 2-hydroxypropyle, méthoxyéthyle, éthoxyéthyle, méthoxypropyle, isobornyle, phényle, 2-phényléthyle, t-butylbenzyle, benzyle, furfurylméthyle ou tétrahydrofurfurylméthyle, méthoxy-polyoxyéthylène (ou POE-méthyle); POE-béhényle, trifluoroéthyle; diméthylaminoéthyle, diéthylaminoéthyle, diméthylaminopropyle.
  et tout particulièrement les (méth)acrylates de méthyle, d'éthyle, de n-propyle, d'isopropyle, de n-butyle, de t-butyle, de cyclohexyle, de méthoxyéthyle, d'éthoxyéthyle, de trifluoroéthyle, de diméthylaminoéthyle, de diéthylaminoéthyle, de 2-hydroxypropyle, de 2-hydroxyéthyle;

- l'acide méthacrylique, le (méth)acrylamide, le méthacryloxypropyl triméthoxy silane, le méthacryloxypropyl tris( triméthylsiloxy)silane;
- ainsi que leurs sels; et leurs mélanges.

Le polymère séquencé selon l'invention comprend en outre une deuxième séquence, appelée ci-après séquence B.
Dans un mode de réalisation préféré de l'invention, le polymère séquencé est un polymère dibloc AB qui comprend, outre la séquence A ci-dessus décrite, une seule séquence supplémentaire, la séquence B.
Dans un autre mode de réalisation préféré de l'invention, le polymère séquencé est un polymère tribloc qui comprend, outre la séquence A ci-dessus décrite, une séquence supplémentaire, ci-après séquence B, et

(i) soit une séquence comprenant à la fois des motifs acrylate d'isobutyle et acide acrylique, c'est-à-dire une séquence A', identique ou différente de ladite séquence A, généralement identique; le polymère séquencé étant au final un polymère tribloc de structure ABA', généralement ABA;
(ii) soit une séquence ne comprenant pas à la fois des motifs acrylate d'isobutyle et acide acrylique, c'est-à-dire une séquence B', identique ou différente de ladite séquence B, généralement identique; le polymère séquencé étant au final un polymère tribloc de structure BAB', généralement BAB.

De préférence, la ou les séquences supplémentaires B, et B' le cas échéant, comprennent principalement des monomères apportant au polymère final la solubilité dans les milieux non aqueux.
Ainsi, la séquence B comprend de préférence 70 à 100% en poids, notamment 75 à 90% en poids, voire 80 à 85% en poids, de monomères, seuls ou en mélange, choisis parmi les monomères solubles.
Par monomère soluble, on entend dans la présente description, tout monomère dont l'homopolymère est sous forme soluble c'est-à-dire complètement dissous et formant une solution limpide, à une concentration de 1% en poids, à température ambiante (25°C, 1 atm), dans l'isododécane.
Les monomères solubles susceptibles d'être employés peuvent être de préférence choisi parmi les monomères solubles suivants, seul ou en mélange, :

- les méthacrylates de formule $CH_2=C(CH_3)-COOR_1$ dans laquelle $R_1$ représente un groupe alkyle linéaire ou ramifié en C4-C22 tel que isobutyle, tertiobutyle, éthyl-2 hexyle, lauryle, béhényle ou stéaryle; ou bien un groupe alkyle cyclique ayant 8 à 30 atomes de carbone, tel que isobornyle;
- les acrylates de formule $CH_2=CH-COOR_2$ dans laquelle $R_2$ représente un groupe alkyle linéaire ou ramifié en C6-C22 tel que éthyl-2 hexyle, lauryle, béhényle ou stéaryle; ou bien un groupe alkyle cyclique ayant 8 à 30 atomes de carbone, tel que isobornyle;
- les (méth)acrylamides de formule $CH_2=C(CH_3)-CONR_3R_4$ ou $CH_2=CH-CONR_3R_4$, dans lesquelles $R_3$ représente un atome d'hydrogène ou un groupe alkyle linéaire ou ramifié en C1-C12, et $R_4$ représente un groupe alkyle en C8-C12 linéaire ou ramifié, tel qu'un groupe isooctyle, isononyle, undecyle;
- les esters de vinyle de formule $R_5-CO-O-CH=CH_2$ où $R_5$ représente un groupe alkyle en $C_8$ à $C_{22}$ linéaire ou ramifié ;
- les éthers d'alcool vinylique et d'alcool de formule $R_6O-CH=CH_2$ dans laquelle $R_6$ représente un groupe alkyle linéaire ou ramifié, comprenant de 8 à 22 atomes de carbone;
- les monomères éthyléniques dont le groupement ester contient des silanes ou des siloxanes, et ne contenant qu'un atome de silicium tel que le (méth)acryloxypropyl trimethoxysilane;
- des macromonomères carbonés ayant un groupe terminal polymérisable;

On entend par "macromonomère ayant un groupe terminal polymérisable" tout oligomère comportant sur une seule de ses extrémités un groupe terminal polymérisable apte à réagir lors de la réaction de polymérisation avec des monomères éthyléniques. Le groupe polymérisable du macromonomère peut être avantageusement un groupe à insaturation éthylénique susceptible de se polymériser par voie radicalaire. Ledit groupe terminal polymérisable peut être en particulier un groupe vinyle ou (méth)acrylate (ou (méth)acryloxy), et de préférence un groupe (méth)acrylate. Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques. Comme macromonomères carbonés ayant un groupe terminal polymérisable, on peut en particulier citer :

- (i) les homopolymères et les copolymères (méth)acrylate d'alkyle linéaire ou ramifié en C6-C22, de préférence en C8-C18, présentant un groupe terminal polymérisable choisi parmi les groupes vinyle ou (méth)acrylate parmi lesquels on peut citer en particulier: les macromonomères de poly(acrylate d'éthyl-2 hexyle) à extrémité mono(méth)acrylate; les macromonomères de poly(acrylate de dodécyle) ou de poly(méthacrylate de dodécyle) à

extrémité mono(méth)acrylate ; les macromonomères de poly(acrylate de stéaryle) ou de poly (méthacrylate de stéaryle) à extrémité mono(méth)acrylate.

De tels macromonomères sont notamment décrits dans les brevets EP895467 et EP96459 et dans l'article Gillman , Polymer Letters, Vol 5, page 477-481 (1967). On peut en particulier citer les macromonomères à base de poly(acrylate d'éthyl-2-hexyle) ou de poly(acrylate de dodécyle) à extrémité mono(méth)acrylate. -(ii) les polyoléfines ayant un groupe terminal à insaturation éthylénique, en particulier ceux ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène ;

De tels macromonomères sont en particulier décrits dans EP 1347013 ou encore dans US 5,625,005 qui mentionne des macromonomères éthylène/butylène et éthylène/propylène à groupement terminal réactif (méth)acrylate. On peut en particulier citer le méthacrylate de poly(éthylène/butylène), tel que celui commercialisé sous la dénomination Kraton Liquid L-1253 par Kraton Polymers.

Comme monomère soluble particulièrement préféré, on peut citer, seuls ou en mélange :

- les méthacrylates de formule $CH_2$=C($CH_3$)-$COOR_1$ dans laquelle $R_1$ représente un groupe alkyle linéaire ou ramifié en C4-C22 ou bien un groupe alkyle cyclique ayant 8 à 30 atomes de carbone;
- les acrylates de formule $CH_2$ = CH-$COOR_2$ dans laquelle $R_2$ représente un groupe alkyle linéaire ou ramifié en C6-C22 ou bien un groupe alkyle cyclique ayant 8 à 30 atomes de carbone.

Les monomères solubles particulièrement préférés sont le (méth)acrylate d'éthyle-2-hexyle, le (méth)acrylate d'isobornyle et le méthacrylate d'isobutyle, et leurs mélanges.

La séquence B peut comprendre de monomères additionnels, qui sont donc non solubles, qui peuvent être présents à raison de 0 à 30% en poids, notamment de 10 à 25% en poids, voire 15 à 20% en poids, dans ladite séquence.

Ces monomères additionnels sont de préférence choisis parmi, seul ou en mélange, les monomères non solubles suivants ainsi que leurs sels :

(i) les (méth)acrylates de formule $CH_2$=CHCOOR ou $CH_2$=C($CH_3$)COOR dans laquelle R représente :

- un groupe alkyle linéaire ou ramifié, comprenant 1 à 30 atomes de carbone dans lequel se trouve(nt) éventuellement intercalé(s) un ou plusieurs hétéroatomes choisis parmi O, N, S et P et/ou ledit groupe alkyle pouvant être éventuellement substitué par un ou plusieurs substituants choisis parmi OH, les atomes d'halogène (Cl, Br, I et F), les groupes -NR4R5, où R4 et R5 identiques ou différents représentent l'hydrogène ou un groupe alkyle linéaire ou ramifié, en C1-C6 ou un groupe phényle; et/ou les groupes polyoxyalkylènes, notamment polyoxyéthylène et/ou polyoxypropylène, ledit groupe polyoxyalkylène étant constitué par la répétition de 5 à 30 motifs oxyalkylène;
- un groupe cycloalkyle en C3-C12, ledit groupe cycloalkyle pouvant comporter dans sa chaîne un ou plusieurs hétéroatomes choisis parmi O, N, S et/ou P, et/ou pouvant être éventuellement substitué par un ou plusieurs substituants choisis parmi -OH et les atomes d'halogène (Cl, Br, I et F);
- un groupe aryle en C4-C20 ou aralkyle en C5-C30 (groupe alkyle en C1-C8);

(ii) les (méth)acrylamides de formule $CH_2$=$CHCONR_1R_2$ ou $CH_2$=C($CH_3$)$CONR_1R_2$ dans laquelle R1 et R2, identiques ou différents, représentent H ou :

- un groupe alkyle, linéaire ou ramifié, comportant de 1 à 18 atomes de carbone, dans lequel se trouve(nt) éventuellement intercalé(s) un ou plusieurs hétéroatomes choisis parmi O, N, S et P; ledit groupe alkyle pouvant être substitué par un ou plusieurs substituants choisis parmi OH et les atomes d'halogène (Cl, Br, I et F), et/ou pouvant être interrompu par un groupe SiR4R5, où R4 et R5 identiques ou différents représentent un groupe alkyle en C1-C6 ou un groupe phényle;
  et notamment un groupe méthyle, éthyle, propyle, isopropyle, n-butyle, isobutyle, tertiobutyle, hexyle, isohexyle, cyclohexyle, éthylhexyle, octyle, isooctyle, décyle, isodécyle, cyclodécyle, dodécyle, cyclododécyle, isononyle, lauryle, t-butyl cyclohexyle, stéaryle; éthyl-2-perfluorohexyle; ou un groupe hydroxyalkyle en C1-4 tel que 2-hydroxyéthyle, 2-hydroxybutyle et 2-hydroxypropyle; ou un groupe alcoxy(C1-4)-alkyle(C1-4) tel que méthoxyéthyle, éthoxyéthyle et méthoxy-propyle,

- un groupe cycloalkyle en C3-C12, tel que isobornyle, ou un groupe hétérocycloalkyle (alkyle en C1-C4), tel que furturylméthyle ou tétrahydrofurfurylméthyle,
- un groupe aryle en C4-C20 tel que le groupe phényle,
- un groupe aralkyle en C5-C30 (groupe alkyle en C1-C8) tel que 2-phényl éthyle, t-butylbenzyle ou benzyle,

(iii) les monomères à insaturation(s) éthylénique(s) comprenant au moins une fonction acide carboxylique, phosphorique ou sulfonique, ou anhydride, comme par exemple l'acide méthacrylique, l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylamidopropanesulfonique; et les sels de ceux ci;

(iv) les éthers de vinyle de formule $R_6O\text{-}CH{=}CH_2$ ou les esters de vinyle de formule : $R_6\text{-}COO\text{-}CH{=}CH_2$ dans laquelle $R_6$ représente un groupe alkyle linéaire ou ramifié, comprenant de 1 à 22 atomes, ou un groupe alkyle cyclique comportant de 3 à 6 atomes de carbone et/ou un groupe aromatique, par exemple de type benzénique, anthracénique, et naphtalénique ;

(v) les composés vinyliques de formules $CH_2{=}CH\text{-}R9$, $CH_2{=}CH\text{-}CH_2\text{-}R9$ ou $CH_2{=}C(CH_3)\text{-}CH_2\text{-}R9$ dans lesquelles R9 est :

- un groupe hydroxyle, halogène (Cl ou F), $NH_2$, acétamide ($NHCOCH_3$);
- un groupe OCOR11 où R11 représente un groupe alkyle en C2-C12, linéaire ou ramifié;
- un groupe OR10 où R10 représente un groupe phényle ou un groupe alkyle en C1-C12;
- un groupe alkyle linéaire ou ramifié, en C1-C18, dans lequel se trouve(nt) éventuellement intercalé(s) un ou plusieurs hétéroatomes choisis parmi O, N, S et P; ledit groupe alkyle pouvant être substitué par un ou plusieurs substituants choisis parmi OH et les atomes d'halogène (Cl, Br, I et F), et/ou pouvant être interrompu par un groupe SiR4R5, où R4 et R5 identiques ou différents représentent un groupe alkyle en C1-C6 ou un groupe phényle;
- un groupe cycloalkyle en C3-C12 tel que isobornyle, cyclohexane,
- un groupe aryle en C3-C20 tel que phényle,
- un groupe aralkyle en C4-C30 (groupe alkyle en C1-C8) tel que 2-phényléthyle ; benzyle,
- un groupe hétérocyclique de 4 à 12 chaînons contenant un ou plusieurs hétéroatomes choisis parmi O, N, et S, le cycle étant aromatique ou non,
- un groupe hétérocycloalkyle (alkyle en C1-C4), tel que furfurylméthyle ou tétrahydrofurfurylméthyle,

(vi) le styrène et ses dérivés, notamment tels que le méthylstyrène, le chlorostyrène ou le chlorométhylstyrène;

(vii) les monomères à insaturation éthylénique comprenant au moins deux atomes de silicium tels que le méthacryloxypropyl tris(triméthylsiloxy)silane.

Parmi ces monomères additionnels, on peut tout particulièrement citer l'acrylate d'isobutyle, le (méth)acrylate de méthyle, l'acide méthacrylique et le méthacryloxypropyl tris(triméthylsiloxy)silane.
La séquence, ou les séquences, comprenant à la fois l'acrylate d'isobutyle et l'acide acrylique (séquence A et éventuellement A') représente(nt) au total de préférence 5 à 55% en poids, notamment 10 à 50% en poids, voire 15 à 47% en poids, encore mieux 20 à 44% en poids, du poids total du polymère séquencé.
La séquence supplémentaire, ou les séquences supplémentaires (séquence B et éventuellement B') représente(nt) au total de préférence 45 à 95% en poids, notamment 50 à 90% en poids, voire 53 à 85% en poids, encore mieux 56 à 80% en poids, du poids total du polymère séquencé.
De préférence, le polymère selon l'invention présente un module E' mesuré par DMTA supérieur ou égal à 550 MPa. L'appareillage employé est un DMA-2980 de TA Instrument; la fréquence est de 1 Hz, la vitesse de balayage est de 3°C par minute, entre -100°C et +200°C.
De préférence, le polymère selon l'invention possède deux températures de transition vitreuse.
De préférence, la séquence A comprenant l'acrylate d'isobutyle et l'acide acrylique, ou l'ensemble de ces séquences si plusieurs, présente une masse moléculaire moyenne en nombre (Mn) comprise entre 5 000 et 50 000 g/mol, de préférence entre 7000 et 45 000.
De préférence, la séquence B ne comprenant pas le mélange acrylate d'isobutyle + acide acrylique, ou l'ensemble de ces séquences si plusieurs, présente une masse moléculaire moyenne en nombre (Mn) comprise entre 50 000 et 150 000 g/mol, de préférence entre 55 000 et 120 000, voire entre 60 000 et 100 000.
De préférence, le polymère séquencé selon l'invention présente un indice de polydispersité (Ip=Mw/Mn) compris entre 1,01 et 2,2.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

Les polymères selon l'invention peuvent être préparés selon les méthodes connues de l'homme du métier. Un procédé général de préparation est décrit avant les exemples.

Le polymère séquencé selon l'invention est préférentiellement soluble dans un milieu non aqueux.

Par 'soluble', on entend que le polymère ne forme pas de précipité, mais une solution limpide, dans ledit milieu non aqueux, à 25°C, 1atm., à une concentration de 1% en poids, dans ledit milieu non aqueux; de préférence à une concentration de 5% en poids, voire de 10% en poids.

Ledit milieu non aqueux comprend, de préférence exclusivement, un ou plusieurs composés non aqueux, liquides à 25°C, ayant un paramètre de solubilité global selon l'espace de solubilité de HANSEN inférieur ou égal à 20 $(MPa)^{1/2}$, ou un mélange de tels composés.

Le paramètre de solubilité global $\delta$ selon l'espace de solubilité de HANSEN est défini dans l'article "Solubility parameter values" de Grulke, dans l'ouvrage "Polymer Handbook" 3ème édition, Chapitre VII, pages 519-559 par la relation :

$$\delta = ( d_D^2 + d_P^2 + d_H^2)^{1/2}$$

dans laquelle :

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.).

La définition des solvants dans l'espace de solubilité tridimensionnel selon HANSEN est décrite dans l'article de HANSEN : "The three dimensional solubility parameters" J. Paint Technol. 39, 105 (1967).

Parmi les composés liquides non aqueux ayant un paramètre de solubilité global selon l'espace de solubilité de HANSEN inférieur ou égal à 20 $(MPa)^{1/2}$, on peut citer les corps gras liquides, notamment les huiles, qui peuvent être choisies parmi les huiles naturelles ou synthétiques, carbonées, voire hydrocarbonées, éventuellement fluorées, éventuellement ramifiées, seules ou en mélange.

On peut notamment citer, seuls ou en mélange :

- les huiles végétales formées par des esters d'acides gras et de polyols, en particulier les triglycérides, telles que l'huile de tournesol, de sésame ou de colza, de macadamia, de soja, l'huile d'amande douce, de calophyllum, de palme, de pépins de raisin, de maïs, d'arara, de coton, d'abricot, d'avocat, de jojoba, d'olive ou de germes de céréales;
- les esters linéaires, ramifiés ou cycliques, ayant 5 à 30 atomes de carbone; et plus particulièrement les esters de formule RCOOR' dans laquelle R représente le reste d'un acide carboxylique comportant 2 à 19 atomes de carbone et R' représente une chaîne hydrocarbonée comportant de 3 à 20 atomes de carbone, tels que les acétates, les palmitates, les adipates, les myristates et les benzoates, notamment l'adipate de diisopropyle et le myristate d'isopropyle;
- les hydrocarbures et notamment les alcanes linéaires, ramifiés et/ou cycliques, volatils ou non volatils, en C5-C60, comme les isoparaffines en C5-C60 éventuellement volatiles tels que l'isododécane, le Parléam (polyisobutène hydrogéné), l'isohexadécane, le cyclohexane, ou les 'ISOPARs'; ou encore les huiles de paraffine, de vaseline, ou le polyisobutylène hydrogéné.
- les éthers ayant 6 à 30 atomes de carbone;
- les cétones ayant 6 à 30 atomes de carbone.
- les monoalcools aliphatiques ayant 6 à 30 atomes de carbone, la chaîne hydrocarbonée ne comportant pas de groupement de substitution, tels que l'alcool oléique, le décanol, le dodécanol, l'octadécanol, l'octyldodécanol et l'alcool linoléique;
- les polyols notamment ayant 6 à 30 atomes de carbone, tels que l'hexylène glycol.

De préférence, le milieux non aqueux comprend, seul ou en mélange, de l'isododécane, du Parléam, de l'acétate de butyle, de l'isononanoate d'isononyle et/ou de l'octyldodécanol.

Les polymères séquencés selon l'invention peuvent être utilisés dans une composition cosmétique qui comprend par ailleurs un milieu cosmétiquement acceptable. Ils peuvent être présents, seuls ou en mélange, dans les compositions cosmétiques selon l'invention en une quantité de 0,1 à 50% en poids, de préférence 0,5 à 40% en poids,

notamment 1 à 30% en poids, de matière sèche par rapport au poids total de la composition.

La composition peut alors comprendre, selon l'application envisagée, les constituants habituels à ce type de composition.

De préférence, ladite composition comprend au moins un milieu non aqueux tel que défini ci-dessus.

Elle peut comprendre en outre d'autres constituants notamment choisis parmi les cires, les huiles, les gommes et/ou les corps gras pâteux, d'origine végétale, animale, minérale ou de synthèse, voire siliconés, et leurs mélanges.

Parmi les cires susceptibles d'être présentes dans la composition selon l'invention, on peut citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane.

La composition selon l'invention peut également comprendre des huiles d'origine minérale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée. Parmi les huiles susceptibles d'être présentes dans la composition selon l'invention, on peut citer, seules ou en mélange, les huiles hydrocarbonées telles que l'huile de paraffine ou de vaseline; le perhydrosqualène; l'huile d'arara; l'huile d'amande douce, de calophyllum, de palme, de ricin, d'avocat, de jojoba, d'olive ou de germes de céréales; des esters d'acide lanolique, d'acide oléique, d'acide laurique, d'acide stéarique; des alcools tels que l'alcool oléique, l'alcool linoléique, l'alcool linolénique, l'alcool isostéarique ou l'octyldodécanol. On peut également citer les huiles siliconées telles que les PDMS, éventuellement phénylées telles que les phényltriméthicones. On peut également utiliser des huiles volatiles, telles que la cyclotétradiméthylsiloxane, la cyclopentadiméthylsiloxane, la cyclohexadiméthylsiloxane, le méthylhexyldiméthylsiloxane, l'hexaméthyldisiloxane ou les isoparaffines.

La composition selon l'invention peut également comprendre une ou plusieurs matières colorantes choisies parmi les composés pulvérulents et/ou les colorants liposolubles ou hydrosolubles.

Les composés pulvérulents peuvent être choisis parmi les pigments et/ou les nacres et/ou les charges habituellement utilisés dans les compositions cosmétiques ou pharmaceutiques. Avantageusement, les composés pulvérulents représentent 0,1 à 50% du poids total de la composition et mieux de 1 à 40%.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon et de polyéthylène, de poly-β-alanine et de polyéthylène, le Téflon, la lauroyllysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium. Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter 0,01 à 20% du poids de la compositions, mieux 0,1 à 6% en poids. Les colorants hydrosolubles sont par exemple le jus de betterave, le bleu de méthylène et peuvent représenter jusqu'à 6% du poids total de la composition.

La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des céramides, des filtres solaires, des polymères, des épaississants, des gélifiants, des tensioactifs. Bien entendu l'homme du métier veillera à choisir ce ou ces additifs et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'une suspension, une dispersion notamment d'huile dans de l'eau grâce à des vésicules; une solution huileuse éventuellement épaissie voire gélifiée; une émulsion

huile-dans-eau, eau-dans-huile, ou multiple; un gel ou une mousse; un gel huileux ou émulsionné; une dispersion de vésicules notamment lipidiques; une lotion biphase ou multi-phase; un spray; d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres, des cils, des ongles; d'un produit solaire ou autobronzant, d'un produit capillaire notamment pour le soin, le maquillage, le maintien, la mise en forme, le nettoyage, le conditionnement, la coloration, le défrisage, des cheveux.

La composition selon l'invention trouve une application particulièrement intéressante dans le domaine du maquillage, plus particulièrement du maquillage des lèvres, des cils, des ongles et/ou du visage. Elle peut donc se présenter avantageusement sous forme de composition de maquillage, notamment de mascara, d'eye-liner, de rouge à lèvres, de fard à joues, de fard à paupières, de fond de teint, de vernis à ongles, de composition de soin du visage, de composition solaire.

L'invention a encore pour objet un procédé de traitement cosmétique des matières kératiniques, notamment de la peau du corps ou du visage, des lèvres, des cils, des ongles et/ou des cheveux, comprenant l'application sur lesdites matières d'une composition cosmétique telle que définie précédemment.

Ce procédé permet notamment le maquillage de la peau, des lèvres, des cils, des ongles et/ou des cheveux, préférentiellement de la peau, des lèvres, des ongles et/ou des cils.

L'invention est illustrée plus en détails dans les exemples suivants.

Le procédé de polymérisation peut être décrit de la manière générale suivante :

Le mélange de monomères, d'amorceur et d'agent RAFT est mis en solution dans un solvant, puis transféré dans un tube de 250 ml. La solution est dégazée par 3 cycles de gel / dégel sous vide (vide de 0,005 mmHg). Le tube est scellé et plongé dans un bain thermostaté pour la durée indiquée. Après la polymérisation, on rajoute du solvant pour dissoudre le polymère, et la solution de polymère est précipitée dans un non-solvant pour récupérer le polymère.

<u>Synthèse des agents RAFT</u>

Bibliographie : Australian journal of chemistry, 2009, vol 62, n°11, pages 391 schéma 11 et page 1453 schéma 14.

a) Agent RAFT ST902-33 : acide 4-cyano-4-(dodecylsulfanylthiocarbonyl)sulfanyl pentanoïque (Polymer 2005, 46, 8458-8468)

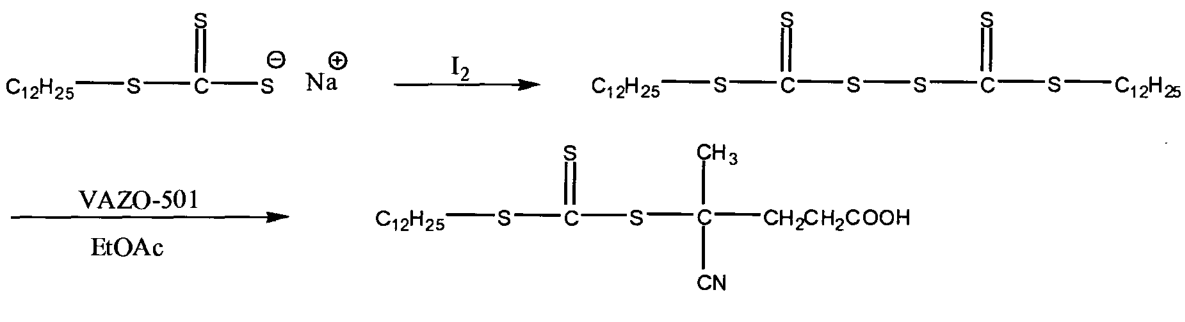


VAZO-501 = 4,4'-azobis(4-cyanopentanoic acid)

On mélange du n-dodecylthiol (15.4 g, 76 mmol) à une suspension de sodium hy-dride (60% dans l'huile) (3.15 g, 79 mmol) dans 150 ml de diéthyléther, à 5-10°C. Le mélange est refroidit à 0°C et on ajoute du carbone disulfide (6.0 g, 79 mmol) afin d'obtenir un précipité de sodium S-dodecyl trithiocarbonate, qui est récupéré par filtration et utilisé dans l'étape suivante, sans purification.

On ajoute de l'iode solide (6.3 g, 0.025 mol) peu à peu, à une suspension de sodium S-dodecyl trithiocarbonate (14.6 g, 0.049 mol) dans 100 ml de diéthyléther. On mélange à 25°C pendant 1 heure, et on élimine par filtration le iodure de sodium jaune formé.

Le filtrat jaune-brun est lavé avec une solution aqueuse de sodium thiosulfate afin d'éliminer l'iode en excès, puis séché sur sodium sulfate, et évaporé de manière à obtenir du bis-(dodecylsulfanylthiocarbonyl) disulfide (13.6 g),

Point de fusion : 33-35°C.

$^1H$ NMR ($CDCl_3$): $\delta$ (ppm) 0.89, t, 6H; 1.30, br s, 36H; 1.71, m, 4H; 3.29, t, 4H.

On chauffe une solution de 4,4'-azobis(4-cyanopentanoïque acide) (2.10 g, 0.0075 mol) et de bis-(dodecylsulfanylthiocarbonyl) disulfide (2.77 g, 0.005 mol) dans 50 ml d'acétate d'éthyle, au reflux pendant 18 heures. On élimine les volatiles sous vide, puis on extrait le produit brut avec de l'eau afin d'obtenir de l'acide 4-cyano-4-(dodecylsulfanylthiocarbonyl) sulfanyl pentanoïque, sous forme de solide jaune pâle (3.65g, rendement : 87%).

Point de fusion : 58-59°C, après recristallisation à l'hexane.

$^1H$ NMR ($CDCl_3$) : $\delta$ (ppm) 0.89 (t, 3H, $CH_3$); 1.28 (br s, 18H); 1.72 (m, 2H); 1.89 (s, 3H, $CH_3$); 2.40-2.80 (m, 4H, $CH_2CH_2$); 3.38 (t, 2H, $CH_2S$).

b) Agent RAFT : ST902-57A

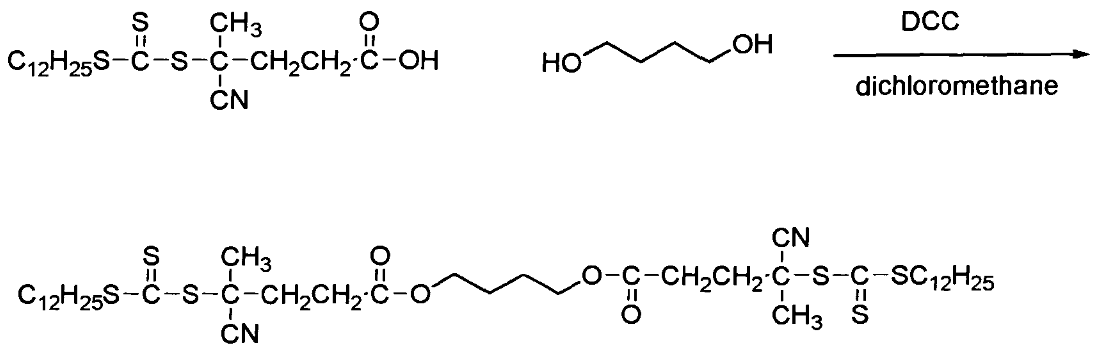


On mélange de l'acide 4-cyano-4-(dodecylsulfanylthiocarbonyl) sulfanyl pentanoï-que (8.1g, 20.1mmol), du 1,4-butanediol (0.9g, 10mmol), du dicyclohexylcarbodii-mide (4.95g, 24.0 mmol) dans 60 ml de dichlorométhane, en présence de DMAP (N,N-dimethylaminopyridine, quantité catalytique), pendant 1 heure à 25°C. On élimine le solvant et le produit brut est passé sur colonne de chromatographie sur silice, avec un mélange éthyl acétate/n-hexane 2/5 comme éluant; on obtient 6,2 g de produit recherché (rendement 72%), sous forme d'une huile jaune qui solidifie au froid.

$^1H$ NMR ($CDCl_3$) : conforme.

c) Agent-RAFT : GXL090612

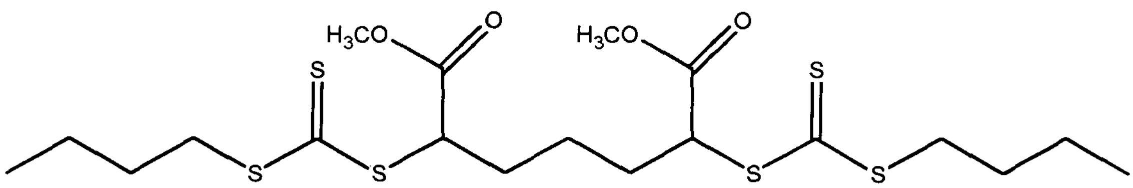

Composé connu : A.M. Bivigou-Koumba, J. Kristen, A. Laschewsky, P. Müller-Buschbaum, C.M. Papadakis. Macromol. Chem. Phys. 2009, 210, 565-578.

__Exemple__ 1

On prépare un polymère-dibloc avec un bloc principal (acrylate d'isobornyle/méthacrylate d'isobornyle 35/35) et un bloc secondaire (acrylate d'isobutyle/Acide acrylique 25/5).

Bloc principale : Mn théorique: 70 000 g/mol

Bloc secondaire : Mn théorique: 30 000 g/mol

L'agent RAFT utilisé est le ST902-33 de formule:

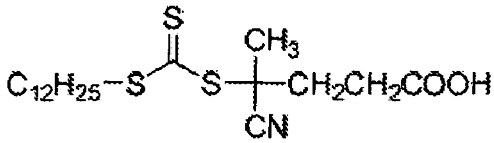

RAFT agent (I) - ST902-33; $C_{19}H_{33}NO_2S_3$ ; MW 403

L'amorceur utilisé est l'AIBN (azobisisobutyronitrile).

Bloc 1 :

- Méthacrylate d'isobornyle 35,01 g
- Acrylate d'isobornyle 35,01 g
- AIBN 101,8 mg
- Agent RAFT 208 mg
- Solvant 30 ml

Conditions réactionnelles : 6 heures à 55°C.

Le solvant est l'acétate d'éthyle (30 ml).

Le non-solvant est le chloroforme (200 ml).

On obtient un premier bloc (57g), rendement 81,2%, de Mn= 71200 g/mol et d'Ip = 1,3.

Polymère dibloc :

- bloc 1 25,0 g
- acrylate d'isobutyle 16,1 g
- acide acrylique 3,2 g
- AIBN 33,1 mg
- Solvant 75 ml + 250 ml

Conditions réactionnelles : 16 heures à 60°C.

Le solvant est le benzène (75 ml) dilué ensuite avec du THF (250 ml).

Le non-solvant est l'acétonitrile (2,5 litres).

On obtient le copolymère dibloc recherché (38,2g), rendement 86,5%, de Mn= 110200 g/mol et d'Ip = 1,5.

Module E' 1 Hz = 816 MPa

Tg = 62°C et 180°C

**Exemple** 2

On prépare un polymère-tribloc avec un bloc central (acrylate d'isobornyle/méthacrylate d'isobornyle 35/35) et deux blocs externes identiques (acrylate d'isobutyle/acide acrylique 12,5/2,5 pour chaque bloc).
Bloc central : Mn théorique: 70 000 g/mol
Blocs externes : Mn théorique: 15 000 g/mol (x 2)

L'agent RAFT utilisé est le ST902-57A.

L'amorceur utilisé est l'AIBN (azobisisobutyronitrile).

Bloc central :

- Méthacrylate d'isobornyle 35,01 g
- Acrylate d'isobornyle 35,03 g
- AIBN 100 mg
- Agent RAFT 430 mg

- Solvant 30 ml

Conditions réactionnelles : 5 heures et 45 minutes à 55°C.
Le solvant est l'acétate d'éthyle (30 ml).
Le non-solvant est le chloroforme (250 ml) et le méthanol (3 litres).
On obtient un premier bloc (54g), rendement : 76,6%, de Mn = 71300 g/mol et d'Ip = 1,2.

Polymère tribloc :

- bloc central 35 g
- acrylate d'isobutyle 13,2 g
- acide acrylique 2,7 g
- AIBN 30 mg
- Solvant 120 ml + 120 ml

Conditions réactionnelles : 22 heures à 60°C.
Le solvant est le chloroforme (120 ml) + le THF (120 ml)
Le non-solvant est l'acétonitrile (2,5 litres).
On obtient le copolymère tribloc recherché (44,1g), rendement 86,6%, de Mn= 96 400 g/mol et d'Ip = 1,2.
Module E' 1 Hz = 720 MPa
Tg = 48°C et 136°C

**Exemple** 3 : comparatif

On prépare un polymère-dibloc avec un bloc principal (acrylate d'isobornyle /méthacrylate d'isobornyle 29/29) et un bloc secondaire (acrylate d'isobutyle 42).
La synthèse se fait en deux étapes: synthèse du bloc polyacrylate d'isobutyle puis du second bloc de poly(acrylate d'isobornyle-co-méthacrylate d'isobornyle).

$1^{ère}$ étape : synthèse du polyacrylate d'isobutyle (Mn= 42 000)

On dispose 150 g d'acrylate d'isobutyle dans un ballon, puis 220μl d'amorceur (éthyl 2-bromoisobutyrate) et 313μl de ligand (PMDETA ou pentaméthyldiéthylene triamine). On introduit le catalyseur (215mg de CuBr(I)) et on laisse sous flux d'argon, dans un bain d'huile à 90°C, pendant 6 heures. La solution est alors versée dans 4 litres d'un mélange 50/50 eau/éthanol, dans lequel le polymère précipite. On élimine la solution eau/EtOH et le polymère est récupéré puis redissout dans du THF. La solution est filtrée sur de l'alumine neutre, puis le solvant est évaporé sous pression réduite

2ème étape: synthèse du polyacrylate d'isobutyle-b-poly(acrylate d'isobornyle-cométhacrylate d'isobornyle)

On introduit 9,6 g de polymère préparé précédemment dans un ballon sous argon, et on ajoute 32,5 ml d'acétate de butyle. On laisse sous agitation jusqu'à complète dissolution. On ajoute 20 g de méthacrylate d'isobornyle, 20 g d'acrylate d'isobornyle, puis 0,048 ml de ligand. On introduit le catalyseur (33 mg de CuBr(I)) et on laisse sous flux d'argon, dans un bain d'huile à 90°C, pendant 52 heures. La solution est alors versée dans 4 litres d'un mélange 50/50 eau/éthanol, dans lequel le polymère précipite. On élimine la solution eau/EtOH et le polymère est récupéré puis redissout dans du THF. La solution est filtrée sur de l'alumine neutre, puis on filtre sur papier et on sèche le polymère obtenu. On obtient le polymère recherché sous forme d'une poudre jaune clair. Mw = 100 000, Ip = 1,3.

**Exemple 4** : **Evaluation**

La tenue à l'huile d'olive est mesurée avec une goutte d'huile d'olive mise sur un film de polymère sec.
Un film de polymère est réalisé à partir de 0,5 ml d'une solution à 20% en poids dans un solvant, étalé sur une plaque de verre de 2,5 x 7,5 cm et séché à température ambiante (25°C) pendant 24 heures. Ensuite, on étale 1 ml d'huile d'olive sur le film de polymère. Après 3 heures, l'excès d'huile est essuyé du film et le collant est appréhendé au toucher.
Exemples 1 et 2 : le solvant est l'acétate de butyle
Exemple comparatif (exemple 3) : le solvant est l'isododécane

On constate que le film formé avec le polymère de l'exemple comparatif (exemple 3) est collant après 3 heures, alors que les films formés avec les polymères des exemples 1 ou 2 sont non collants après 3 heures.

Le collant traduit la sensibilité du polymère à l'huile d'olive. Plus cette sensibilité est importante, plus le dépôt sera facilement altéré lors des repas, engendrant une moins bonne tenue. La tenue des polymères selon l'invention est supérieure à celle du polymère comparatif.

**Exemple 5: composition cosmétique**

On a dissout 25 g du polymère de l'exemple 1 (obtenu sous forme sèche) dans 70 g d'un mélange acétate de butyle/ acétate d'éthyle (70/30 en poids).

La solution obtenue est ensuite appliquée sur les ongles. Le film présente une bonne tenue dans le temps.

**Exemple 6 : composition cosmétique**

On a dissout 25 g du polymère de l'exemple 2 (obtenu sous forme sèche) dans 70 g d'un mélange acétate de butyle/ acétate d'éthyle (70/30 en poids).

La solution obtenue est ensuite appliquée sur les ongles. Le film présente une bonne tenue dans le temps.

**Exemple 7 : composition cosmétique**

On prépare un gloss pour les lèvres ayant la composition suivante (% en poids):

- polybutène 34%
- isononanoate d'isononyle 4%
- octyldodécanol 10%
- silice (Aérosil R972) 5%
- polymère de l'exemple 2 14% MS
- isododécane 20%
- trimellitate de tridécyle qsp 100%

Le mélange obtenu est ensuite appliqué sur les lèvres. Le dépôt présente de bonnes propriétés cosmétiques.

## Revendications

1. Polymère di ou tri-séquencé de structure AB, ABA ou BAB, comprenant au moins une séquence A qui comprend de l'acrylate d'isobutyle et de l'acide acrylique.

2. Polymère selon la revendication 1, se présentant sous la forme d'un polymère éthylénique, de préférence linéaire.

3. Polymère selon l'une des revendications précédentes, dans lequel, dans la séquence A, l'acrylate d'isobutyle représente 75 à 99,5% en poids, notamment 78 à 95% en poids du poids total du mélange acrylate d'isobutyle + acide acrylique; et l'acide acrylique représente 0,5 à 25% en poids, notamment 5 à 22% en poids du poids total du mélange acrylate d'isobutyle + acide acrylique.

4. Polymère selon l'une des revendications précédentes, dans lequel le mélange acrylate d'isobutyle + acide acrylique représente 50 à 100% en poids, notamment 60 à 90% en poids, voire 70 à 80% en poids, du poids total de ladite séquence A.

5. Polymère selon l'une des revendications précédentes, dans lequel ladite séquence A comprend au moins un monomère additionnel choisi parmi, seul ou en mélange, :

   - les (méth)acrylates en C1-C18, comprenant éventuellement un hétéroatome,
   - l'acide méthacrylique, le (méth)acrylamide, le méthacryloxypropyl triméthoxy silane, le méthacryloxypropyl tris(triméthylsiloxy)silane;
   - ainsi que leurs sels.

6. Polymère selon l'une des revendications précédentes, comprenant en outre une deuxième séquence, ou séquence

B, qui comprend 70 à 100% en poids, notamment 75 à 90% en poids, voire 80 à 85% en poids, de monomères, seuls ou en mélange, choisis parmi les monomères solubles suivants, seul ou en mélange, :

- les méthacrylates de formule $CH_2=C(CH_3)-COOR_1$ dans laquelle $R_1$ représente un groupe alkyle linéaire ou ramifié en C4-C22; ou bien un groupe alkyle cyclique ayant 8 à 30 atomes de carbone;
- les acrylates de formule $CH_2=CH-COOR_2$ dans laquelle $R_2$ représente un groupe alkyle linéaire ou ramifié en C6-C22 ou bien un groupe alkyle cyclique ayant 8 à 30 atomes de carbone;
- les (méth)acrylamides de formule $CH_2=C(CH_3)-CONR_3R_4$ ou $CH_2=CH-CONR_3R_4$, dans lesquelles $R_3$ représente un atome d'hydrogène ou un groupe alkyle linéaire ou ramifié en C1-C12, et $R_4$ représente un groupe alkyle en C8-C12 linéaire ou ramifié;
- les esters de vinyle de formule $R_5-CO-O-CH=CH_2$ où $R_5$ représente un groupe alkyle en $C_8$ à $C_{22}$ linéaire ou ramifié ;
- les éthers d'alcool vinylique et d'alcool de formule $R_6OA-CH=CH_2$ dans laquelle $R_6$ représente un groupe alkyle linéaire ou ramifié, comprenant de 8 à 22 atomes de carbone;
- les monomères éthyléniques dont le groupement ester contient des silanes ou des siloxanes, et ne contenant qu'un atome de silicium;
- des macromonomères carbonés ayant un groupe terminal polymérisable;

**7.** Polymère selon la revendication 6, dans lequel les monomères solubles sont choisis parmi les monomères suivants, seul ou en mélange, :

- les méthacrylates de formule $CH_2=C(CH_3)-COOR_1$ dans laquelle $R_1$ représente un groupe alkyle linéaire ou ramifié en C4-C22 ou bien un groupe alkyle cyclique ayant 8 à 30 atomes de carbone;
- les acrylates de formule $CH_2 = CH-COOR_2$ dans laquelle $R_2$ représente un groupe alkyle linéaire ou ramifié en C6-C22 ou bien un groupe alkyle cyclique ayant 8 à 30 atomes de carbone.

**8.** Polymère selon l'une des revendications précédentes, dans lequel la ou les séquences comprenant à la fois l'acrylate d'isobutyle et l'acide acrylique représente(nt) au total 5 à 55% en poids, notamment 10 à 50% en poids, voire 15 à 47% en poids, encore mieux 20 à 44% en poids, du poids total du polymère séquencé.

**9.** Polymère selon l'une des revendications précédentes, dans lequel la ou les séquences comprenant l'acrylate d'isobutyle et l'acide acrylique présente(nt) une masse moléculaire moyenne en nombre (Mn) comprise entre 5 000 et 50 000 g/mol, de préférence entre 7000 et 45 000.

**10.** Polymère selon l'une des revendications précédentes, présentant un indice de polydispersité (Ip=Mw/Mn) compris entre 1,01 et 2,2.

**11.** Composition cosmétique comprenant, dans un milieu cosmétiquement acceptable, au moins un polymère selon l'un des revendications précédentes.

**12.** Composition selon la revendication 11, dans laquelle le polymère est présent, seul ou en mélange, en une quantité de 0,1 à 50% en poids, de préférence 0,5 à 40% en poids, notamment 1 à 30% en poids, de matière sèche par rapport au poids total de la composition.

**13.** Composition selon l'une des revendications 11 à 12, dans laquelle le milieu cosmétiquement acceptable comprend au moins un ingrédient choisi parmi les cires, les huiles, les gommes, les corps gras pâteux, d'origine végétale, animale, minérale ou de synthèse, voire siliconés; les matières colorantes choisies parmi les composés pulvérulents et/ou les colorants liposolubles ou hydrosolubles; des antioxydants, des parfums, des huiles essentielles, des conservateurs, des actifs cosmétiques, des hydratants, des vitamines, des acides gras essentiels, des céramides, des filtres solaires, des polymères, des épaississants, des gélifiants, des tensioactifs.

**14.** Composition selon l'une des revendications 11 à 13, se présentant sous la forme d'un produit de soin et/ou de maquillage de la peau du corps ou du visage, des lèvres, des cils, des ongles; d'un produit solaire ou autobronzant, d'un produit capillaire notamment pour le soin, le maquillage, le maintien, la mise en forme, le nettoyage, le conditionnement, la coloration, le défrisage, des cheveux.

**15.** Procédé de traitement cosmétique des matières kératiniques, notamment de la peau du corps ou du visage, des lèvres, des cils, des ongles et/ou des cheveux, comprenant l'application sur lesdites matières d'une composition

cosmétique telle que définie à l'une des revendications 11 à 14.

## Patentansprüche

1. Diblock- oder Triblockpolymer mit AB-, ABA- oder BAB-Struktur, umfassend mindestens einen Block A, der Isobutylacrylat und Acrylsäure umfasst.

2. Polymer nach Anspruch 1 in Form eines ethylenischen Polymers, das vorzugsweise linear ist.

3. Polymer nach einem der vorhergehenden Ansprüche, wobei in Block A das Isobutylacrylat 75 bis 99,5 Gew.-%, insbesondere 78 bis 95 Gew.-%, des Gesamtgewichts der Mischung von Isobutylacrylat + Acrylsäure ausmacht und die Acrylsäure 0,5 bis 25 Gew.-%, insbesondere 5 bis 22 Gew.-%, des Gesamtgewichts der Mischung von Isobutylacrylat + Acrylsäure ausmacht.

4. Polymer nach einem der vorhergehenden Ansprüche, wobei die Mischung von Isobutylacrylat + Acrylsäure 50 bis 100 Gew.-%, insbesondere 60 bis 90 Gew.-% oder sogar 70 bis 80 Gew.-% des Gesamtgewichts des Blocks A ausmacht.

5. Polymer nach einem der vorhergehenden Ansprüche, wobei der Block A mindestens ein zusätzliches Monomer umfasst, das allein oder als Gemisch aus:

   - $C_1$-$C_{18}$-(Meth)acrylaten, die gegebenenfalls ein Heteroatom umfassen,
   - Methacrylsäure, (Meth)acrylamid, Methacryloxypropyltrimethoxysilan, Methacryloxypropyltris(trimethylsiloxy)silan
   - sowie deren Salzen

   ausgewählt ist.

6. Polymer nach einem der vorhergehenden Ansprüche, außerdem umfassend einen zweiten Block oder Block B, der 70 bis 100 Gew.-%, insbesondere 75 bis 90 Gew.-% oder sogar 80 bis 85 Gew.-% Monomere, alleine oder als Gemisch, die aus den folgenden löslichen Monomeren, alleine oder als Gemisch, ausgewählt sind, umfasst:

   - Methacrylaten der Formel $CH_2=C(CH_3)-COOR_1$, in der $R_1$ für eine lineare oder verzweigte $C_4$-$C_{22}$-Alkylgruppe oder auch eine cyclische Alkylgruppe mit 8 bis 30 Kohlenstoffatomen steht;
   - Acrylaten der Formel $CH_2=CH-COOR_2$, in der $R_2$ für eine lineare oder verzweigte $C_6$-$C_{22}$-Alkylgruppe oder auch eine cyclische Alkylgruppe mit 8 bis 30 Kohlenstoffatomen steht;
   - (Meth) acrylamiden der Formel $CH_2=C(CH_3)-CONR_3R_4$ bzw. $CH_2=CH-CONR_3R_4$, in denen $R_3$ für ein Wasserstoffatom oder eine lineare oder verzweigte $C_1$-$C_{12}$-Alkylgruppe steht und $R_4$ für eine lineare oder verzweigte $C_8$-$C_{12}$-Alkylgruppe steht;
   - Vinylestern der Formel $R_5-CO-O-CH=CH_2$, in der $R_5$ für eine lineare oder verzweigte $C_8$- bis $C_{22}$-Alkylgruppe steht;
   - Ethern von Vinylalkohol und Alkohol der Formel $R_6O-CH=CH_2$, in der $R_6$ für eine lineare oder verzweigte Alkylgruppe mit 8 bis 22 Kohlenstoffatomen steht;
   - ethylenischen Monomeren, deren Estergruppe Silane oder Siloxane enthält und die nur ein Siliciumatom enthalten;
   - auf Kohlenstoff basierenden Makromonomeren mit einer polymerisierbaren Endgruppe.

7. Polymer nach Anspruch 6, wobei die löslichen Monomere aus den folgenden Monomeren, alleine oder als Gemisch, ausgewählt sind:

   - Methacrylaten der Formel $CH_2=C(CH_3)-COOR_1$, in der $R_1$ für eine lineare oder verzweigte $C_4$-$C_{22}$-Alkylgruppe oder auch eine cyclische Alkylgruppe mit 8 bis 30 Kohlenstoffatomen steht;
   - Acrylaten der Formel $CH_2=CH-COOR_2$, in der $R_2$ für eine lineare oder verzweigte $C_6$-$C_{22}$-Alkylgruppe oder auch eine cyclische Alkylgruppe mit 8 bis 30 Kohlenstoffatomen steht.

8. Polymer nach einem der vorhergehenden Ansprüche, wobei der Block bzw. die Blöcke, der bzw. die sowohl Isobutylacrylat als auch Acrylsäure umfasst bzw. umfassen, insgesamt 5 bis 55 Gew.-%, insbesondere 10 bis 50 Gew.-% oder sogar 15 bis 47 Gew.-%, noch besser 20 bis 44 Gew.-%, des Gesamtgewichts des Blockpolymers ausmacht

bzw. ausmachen.

**9.** Polymer nach einem der vorhergehenden Ansprüche, wobei der Block bzw. die Blöcke, der bzw. die Isobutylacrylat und Acrylsäure umfasst bzw. umfassen, ein zahlenmittleres Molekulargewicht (Mn) zwischen 5000 und 50.000 g/mol, vorzugsweise zwischen 7000 und 45.000, aufweist bzw. aufweisen.

**10.** Polymer nach einem der vorhergehenden Ansprüche, das einen Polydispersitätsindex (Ip=Mw/Mn) zwischen 1,01 und 2,2 aufweist.

**11.** Kosmetische Zusammensetzung, die in einem kosmetisch unbedenklichen Medium mindestens ein Polymer nach einem der vorhergehenden Ansprüche umfasst.

**12.** Zusammensetzung nach Anspruch 11, in der das Polymer, alleine oder als Gemisch, in einer Menge von 0,1 bis 50 Gew.-%, vorzugsweise 0,5 bis 40 Gew.-%, insbesondere 1 bis 30 Gew.-%, Trockensubstanz, bezogen auf das Gesamtgewicht der Zusammensetzung, vorliegt.

**13.** Zusammensetzung nach einem der Ansprüche 11 bis 12, wobei das kosmetisch unbedenkliche Medium mindestens einen Bestandteil umfasst, der aus Wachsen, Ölen, Gummen, pastösen Fettsubstanzen pflanzlichen, tierischen, mineralischen oder synthetischen oder sogar silikonischen Ursprungs; farbgebenden Stoffen, die aus pulverförmigen Verbindungen und/oder fettlöslichen oder wasserlöslichen Farbstoffen ausgewählt sind; Antioxidantien, Parfümen, etherischen Ölen, Konservierungsmitteln, kosmetischen Wirkstoffen, Feuchtigkeitsspendern, Vitaminen, essentiellen Fettsäuren, Ceramiden, Sonnenschutzmitteln, Polymeren, Verdickungsmitteln, Geliermitteln und Tensiden ausgewählt ist.

**14.** Zusammensetzung nach einem der Ansprüche 11 bis 13, die in Form eines Produkts zur Pflege und/oder zum Make-up der Körper- oder Gesichtshaut, der Lippen, der Wimpern oder der Nägel; eines Sonnenschutz- oder Selbstbräunungsprodukts oder eines Haarprodukts, insbesondere zur Pflege, zum Make-up, der Erhaltung der Form, der Formgebung, der Reinigung, der Konditionierung, der Färbung oder der Entkräuselung der Haare, vorliegt.

**15.** Kosmetisches Behandlungsverfahren für Keratinmaterialien, insbesondere die Körper- oder Gesichtshaut, die Lippen, die Wimpern, die Nägel und/oder die Haare, bei dem man auf die Materialien eine kosmetische Zusammensetzung gemäß einem der Ansprüche 11 bis 14 aufbringt.

## Claims

**1.** Diblock or triblock polymer of AB, ABA or BAB structure, comprising at least one block A which comprises isobutyl acrylate and acrylic acid.

**2.** Polymer according to Claim 1, in the form of an ethylenic polymer, which is preferably linear.

**3.** Polymer according to either of the preceding claims, in which, in the block A, the isobutyl acrylate represents 75% to 99.5% by weight, in particular 78% to 95% by weight, of the total weight of the isobutyl acrylate + acrylic acid mixture; and the acrylic acid represents 0.5% to 25% by weight, in particular 5% to 22% by weight, of the total weight of the isobutyl acrylate + acrylic acid mixture.

**4.** Polymer according to one of the preceding claims, in which the isobutyl acrylate + acrylic acid mixture represents 50% to 100% by weight, in particular 60% to 90% by weight, or even 70% to 80% by weight, of the total weight of said block A.

**5.** Polymer according to one of the preceding claims, in which said block A comprises at least one additional monomer chosen from, alone or as a mixture:

- $C_1$-$C_{18}$ (meth)acrylates, optionally comprising a heteroatom;
- methacrylic acid, (meth)acrylamide, methacryloxypropyltrimethoxysilane, methacryloxypropyltris(trimethylsiloxy)silane;
- and the salts thereof.

6. Polymer according to one of the preceding claims, also comprising a second block, or block B, which comprises 70% to 100% by weight, in particular 75% to 90% by weight, or even 80% to 85% by weight, of monomers, alone or as a mixture, chosen from the following soluble monomers, alone or as a mixture:

- methacrylates of formula $CH_2=C(CH_3)-COO_{R1}$ in which $R_1$ represents a linear or branched $C_4$-$C_{22}$ alkyl group or else a cyclic alkyl group having 8 to 30 carbon atoms;
- acrylates of formula $CH_2=CH-COOR_2$ in which $R_2$ represents a linear or branched $C_6$-$C_{22}$ alkyl group or else a cyclic alkyl group having 8 to 30 carbon atoms;
- (meth)acrylamides of formula $CH_2=C(CH_3)-CONR_3R_4$ or $CH_2=CH-CONR_3R_4$, in which $R_3$ represents a hydrogen atom or a linear or branched $C_1$-$C_{12}$ alkyl group, and $R_4$ represents a linear or branched $C_8$-$C_{12}$ alkyl group;
- vinyl esters of formula $R_5-CO-O-CH=CH_2$ in which $R_5$ represents a linear or branched $C_8$ to $C_{22}$ alkyl group;
- ethers of vinyl alcohol and of alcohol of formula $R_6O-CH=CH_2$ in which $R_6$ represents a linear or branched alkyl group comprising from 8 to 22 carbon atoms;
- ethylenic monomers of which the ester group contains silanes or siloxanes, and containing only one silicon atom;
- carbon-based macromonomers which have a polymerizable end group.

7. Polymer according to Claim 6, in which the soluble monomers are chosen from the following monomers, alone or as a mixture:

- methacrylates of formula $CH_2=C(CH_3)-COOR_1$ in which $R_1$ represents a linear or branched $C_4$-$C_{22}$ alkyl group or else a cyclic alkyl group having 8 to 30 carbon atoms;
- acrylates of formula $CH_2=CH-COOR_2$ in which $R_2$ represents a linear or branched $C_6$-$C_{22}$ alkyl group or else a cyclic alkyl group having 8 to 30 carbon atoms.

8. Polymer according to one of the preceding claims, in which the block, or blocks, comprising both isobutyl acrylate and acrylic acid represent(s) in total 5% to 55% by weight, in particular 10% to 50% by weight, or even 15% to 47% by weight, even better still 20% to 44% by weight, of the total weight of the block polymer.

9. Polymer according to one of the preceding claims, in which the block, or blocks, comprising isobutyl acrylate and acrylic acid has (have) a number-average molecular weight (Mn) of between 5000 and 50 000 g/mol, preferably between 7000 and 45 000.

10. Polymer according to one of the preceding claims, which has a polydispersity index (Ip=Mw/Mn) of between 1.01 and 2.2.

11. Cosmetic composition comprising, in a cosmetically acceptable medium, at least one polymer according to one of the preceding claims.

12. Composition according to Claim 11, in which the polymer is present, alone or as a mixture, in an amount of from 0.1% to 50% by weight, preferably 0.5% to 40% by weight, in particular 1% to 30% by weight, of dry matter relative to the total weight of the composition.

13. Composition according to either of Claims 11 and 12, in which the cosmetically acceptable medium comprises at least one ingredient chosen from waxes, oils, gums, pasty fatty substances, of vegetable, animal, mineral or synthetic origin, or even which are silicone-based; colorants chosen from pulverulent compounds and/or dyes which are liposoluble or watersoluble; antioxidants, fragrances, essential oils, preservatives, cosmetic active agents, moisturizers, vitamins, essential fatty acids, ceramides, sunscreens, polymers, thickeners, gelling agents and surfactants.

14. Composition according to one of Claims 11 to 13, in the form of a product for caring for and/or making up bodily or facial skin, the lips, the eyelashes or the nails; of an anti-sun or self-tanning product, or of a hair product, in particular for caring for, making up, retaining the form of, shaping, cleansing, conditioning, dyeing or straightening the hair.

15. Cosmetic process for treating keratin materials, in particular bodily or facial skin, the lips, the eyelashes, the nails and/or the hair, comprising the application to said materials of a cosmetic composition as defined in one of Claims 11 to 14.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 895467 A
- EP 96459 A
- US 5625005 A
- EP 1347013 A


### Littérature non-brevet citée dans la description


- **GILLMAN.** *Polymer Letters,* 1967, vol. 5, 477-481
- Solubility parameter values. **GRULKE.** Polymer Handbook. 519-559
- **HANSEN.** The three dimensional solubility parameters. *J. Paint Technol.,* 1967, vol. 39, 105
- *Australian journal of chemistry,* 2009, vol. 62 (11), 391
- *Polymer,* 2005, vol. 46, 8458-8468
- **A.M. BIVIGOU-KOUMBA ; J. KRISTEN ; A. LASCHEWSKY ; P. MÜLLER-BUSCHBAUM ; C.M. PAPADAKIS.** *Macromol. Chem. Phys.,* 2009, vol. 210, 565-578